(19) Europäisches Patentamt
European Patent Office
Office européen des brevets

(11) **EP 3 919 032 B1**

(12) **EUROPEAN PATENT SPECIFICATION**

(45) Date of publication and mention
of the grant of the patent:
**04.12.2024 Bulletin 2024/49**

(21) Application number: **20749464.2**

(22) Date of filing: **28.01.2020**

(51) International Patent Classification (IPC):
**A61F 13/0246** (2024.01)   **A61L 15/42** (2006.01)
**A61L 15/58** (2006.01)   **A61L 24/00** (2006.01)
**A61L 24/06** (2006.01)   **C09J 7/26** (2018.01)
**C09J 7/38** (2018.01)   **C08K 5/103** (2006.01)

(52) Cooperative Patent Classification (CPC):
(C-Sets available)
**A61F 13/0253; A61F 13/025; A61L 24/0036;**
**A61L 24/06; C09J 7/26;** C09J 2301/302;
C09J 2301/312; C09J 2400/243; C09J 2433/00

(Cont.)

(86) International application number:
**PCT/JP2020/002878**

(87) International publication number:
**WO 2020/158697 (06.08.2020 Gazette 2020/32)**

(54) **ADHESIVE SKIN PATCH MATERIAL**

HAUTPFLASTERMATERIAL

MATÉRIAU DE TIMBRE TRANSDERMIQUE ADHÉSIF

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **28.01.2019 JP 2019012195**

(43) Date of publication of application:
**08.12.2021 Bulletin 2021/49**

(73) Proprietor: **NITTO DENKO CORPORATION**
**Ibaraki-shi**
**Osaka 567-8680 (JP)**

(72) Inventors:
• **YOSHIDA, Noboru**
**Ibaraki-shi, Osaka 567-8680 (JP)**
• **KODAMA, Kiyoaki**
**Ibaraki-shi, Osaka 567-8680 (JP)**
• **ABE, Eriko**
**Ibaraki-shi, Osaka 567-8680 (JP)**
• **ARIMA, Ryuhei**
**Ibaraki-shi, Osaka 567-8680 (JP)**

(74) Representative: **Grünecker Patent- und**
**Rechtsanwälte**
**PartG mbB**
**Leopoldstraße 4**
**80802 München (DE)**

(56) References cited:
EP-A1- 0 106 440        EP-A1- 3 498 245
WO-A1-2018/030447       DE-A1- 102016 008 257
JP-A- 2015 173 948      JP-A- 2018 075 057
JP-A- H0 724 001        JP-A- H04 308 526
JP-A- H10 328 231       JP-U- H045 807

(52) Cooperative Patent Classification (CPC): (Cont.)

C-Sets
**A61L 24/06, C08L 33/08;**
**A61L 24/06, C08L 33/10**

C-Sets
**A61L 24/06, C08L 33/08;**
**A61L 24/06, C08L 33/10**

**Description**

Technical Field

The present invention relates to an adhesive skin patch.

Background Art

A device (wearable device) that is mounted on a body of a user is conventionally known. The wearable device is versatile, and one example of the use is measurement of information such as blood glucose level, heart rhythm, myoelectricity, body temperature and the like of a user.

Furthermore, there are various manners to mount a wearable device. For example, a device mounting by sticking to a body of a user is known. Such a device is excellent in that due to high adhesiveness to a body of a user, accuracy of information is high, and mounting is simple. For example, Patent Document 1 discloses a device for cardiac monitoring that is used by sticking to a skin.

Tape-like adhesive skin patch having adhesiveness is sometimes used to stick such a device to a skin. Examples of adhesive patches and wound dressings are disclosed in Patent Documents 2 to 4 and Utility Model 1.

Related Art

> Patent Document 1: JP-T-2015-530225 (the term "JP-T" as used herein means a published Japanese translation of a PCT patent application)
> Patent Document 2: DE 10 2016 008257 A1
> Patent Document 3: WO 2018/030447 A1
> Patent Document 4: EP 0 106 440 A1
> Utility Model 1: JP H04 5807 U

Summary of Invention

Problems to Be Solved by the Invention

When an adhesive skin patch is being stuck to a skin, contact surface between the skin and the adhesive skin patch gets stuffy by moisture such as sweat. As a result, adhesiveness is deteriorated, patch may be peeled, discomfort may occur, and in some cases, skin irritation such as an itch or inflammation may occur. In particular, when a wearable device or its structural member is fixed to a skin through the adhesive skin patch, water vapor permeability of the surface of a substrate of the adhesive skin patch is restricted by the presence of the wearable device or the like. As a result, stuffiness is further easy to occur on a skin surface, and the decrease of adhesiveness and the occurrence of discomfort and skin irritation become further remarkable.

In addition, since a skin flexibly stretches, a wearable device and an adhesive skin patch cannot follow expansion and contraction of the skin, and as a result, those may peel from the skin or discomfort (skin tightness) and pain may be felt.

The wearable device may be required to be mounted on the skin over a long period of time depending on its uses. Therefore, an adhesive skin patch that has less skin irritation, is difficult to peel from a skin and suppresses discomfort during sticking to the skin has been desired.

The present invention has been made in view of the above and has an object to provide an adhesive skin patch that has less skin irritation, is difficult to peel from a skin and suppresses discomfort during sticking to a skin.

Means for Solving the Problems

The adhesive skin patch of the present invention that solves the above problems is an adhesive skin patch including a substrate and an adhesive layer, wherein the substrate is formed of a foam sheet having open cells and a bubble fraction of 70% or more, and the adhesive layer is partially formed on one side of the substrate in a wavy line shape.

In one aspect of the adhesive skin patch of the present invention, another adhesive layer may be further provided on a surface of a side opposite the surface of the substrate having the adhesive layer.

In one aspect of the adhesive skin patch of the present invention, the adhesive layer may contain an acrylic copolymer.

In one aspect of the adhesive skin patch of the present invention, the acrylic copolymer may be an acrylic copolymer obtained by copolymerizing a monomer mixture containing a (meth)acrylic acid alkyl ester monomer and an alkoxy group-containing ethylenically unsaturated monomer.

In one aspect of the adhesive skin patch of the present invention, a bubble fraction of the foam sheet may be 70 to 98.5%.

Effects of the Invention

According to the present invention, an adhesive skin patch that has less skin irritation, is difficult to peel from a skin and suppresses discomfort during sticking to a skin is provided.

Brief Description of Drawings

[FIG. 1]

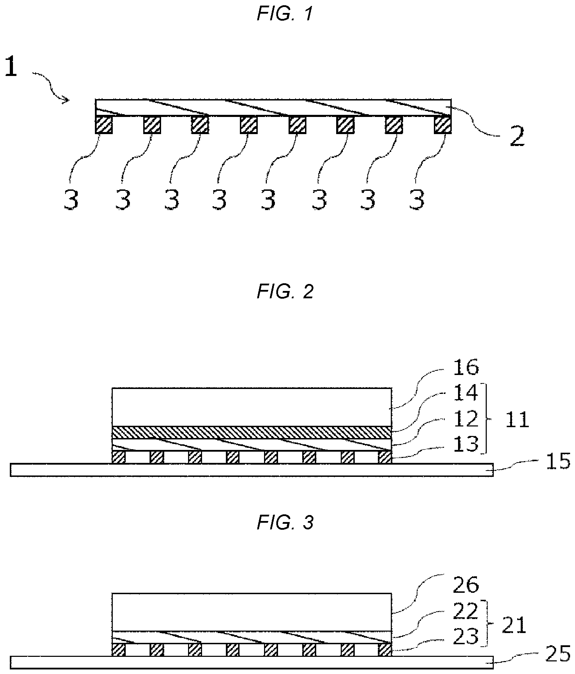

FIG. 1 is a schematic side view of an adhesive skin patch.
[FIG. 2]
FIG. 2 is a schematic view showing the state that a wearable device has been stuck to a skin using an adhesive skin patch in which both sides are an adhesive surface.
[FIG. 3]
FIG. 3 is a schematic view showing the state that a wearable device has been stuck to a skin using an adhesive skin patch in which only one side is an adhesive surface.
[FIG. 4]

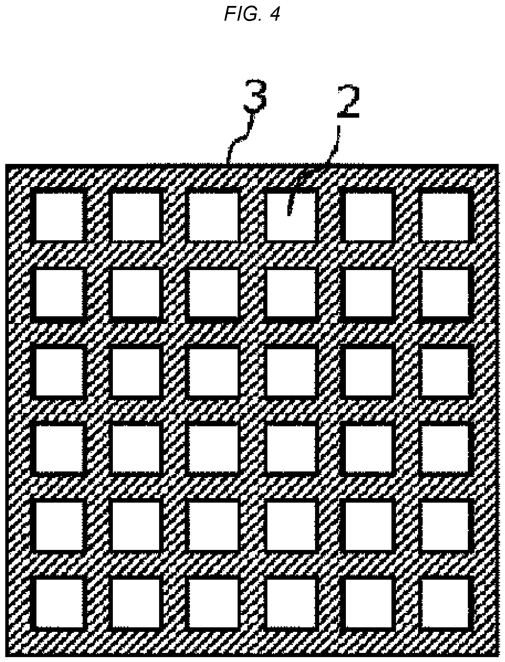

FIG. 4 is a plane view showing one example of a shape of an adhesive layer.
[FIG. 5]

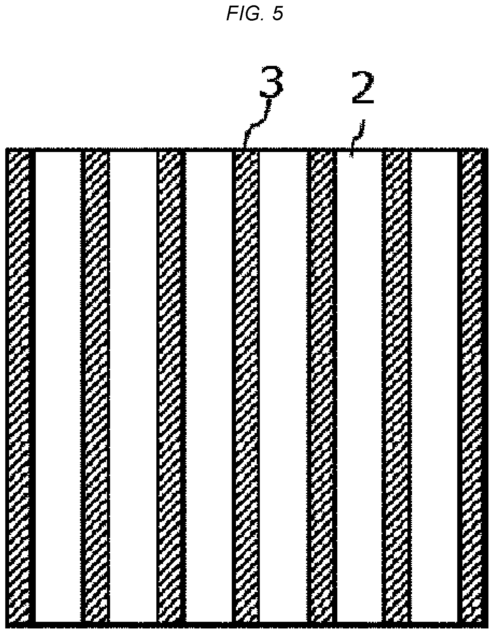

FIG. 5 is a plane view showing one example of a shape of an adhesive layer.
[FIG. 6]

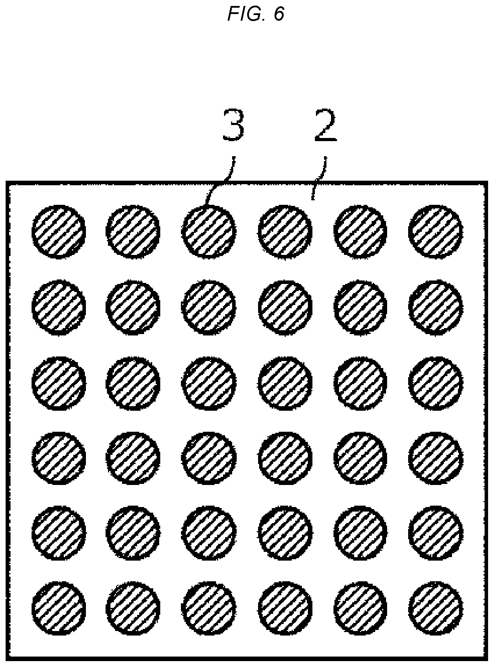

FIG. 6 is a plane view showing one example of a shape of an adhesive layer.
[FIG. 7]

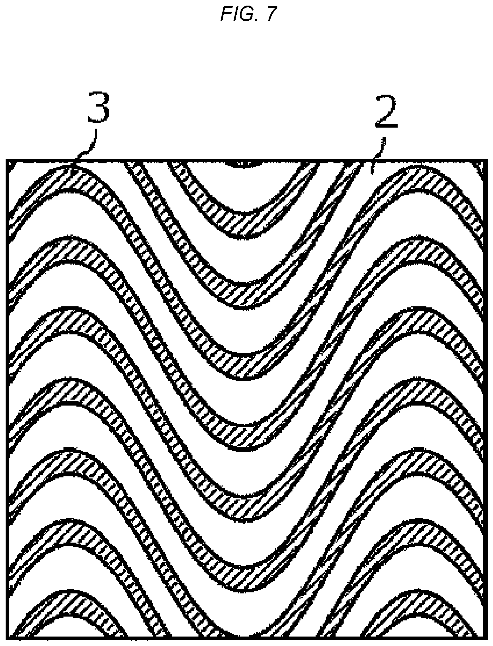

FIG. 7 is a plane view showing one example of a shape of an adhesive layer according to the present invention.

Embodiments for Carrying Out the Invention

The embodiments of the present invention have been described in detail blow. However, the present invention is not construed as being limited to the embodiments described below.

[Adhesive skin patch]

Schematic side view of the adhesive skin patch according to one aspect of the present embodiment is shown in FIG. 1. An adhesive skin patch 1 of the present embodiment is an adhesive skin patch having a substrate (support) 2 and an adhesive layer 3.

In the present embodiment, the substrate 2 is formed of a foam sheet having open cells and a bubble fraction of 70% or more. The open cells indicate bubbles that are connected with other bubbles, among the bubbles in the foam sheet. Among the bubbles in the foam sheet, bubbles that are not connected with other bubbles are called closed cells. All of the bubbles in the substrate 2 may not be open cells. In other words, the substrate 2 may be a form having only open cells (open cell structure) and may be a form having both open cells and closed cells (semi-open and semi-closed cell structure).

In the present embodiment, the adhesive layer 3 is partially formed on one side of the substrate 2 in a wavy line shape. In the following description, the surface of the substrate 2 on the side having the adhesive layer 3 partially formed is sometimes described as a "first surface" and the surface on the side opposite the first surface is sometimes called a "second surface".

The adhesive skin patch 1 of the present embodiment is stuck to a skin through the adhesive layer 3. In other words, the adhesive skin patch 1 of the present embodiment has the portion on which the adhesive layer 3 is not formed, on the surface of the skin side.

In the adhesive skin patch 1 having the above constitution of the present embodiment, moisture such as sweat generated from a skin passes through the portion on which the adhesive layer 3 is not formed, reaches the first surface of the substrate 2, passes through the open cells and moves outside from the edge or second surface of the substrate 2. In other words, the adhesive skin patch 1 of the present embodiment has excellent moisture permeability. Therefore, the adhesive skin patch 1 of the present embodiment suppresses the occurrence of peeling, discomfort and skin irritation due to moisture such as sweat.

From the standpoint of improvement in stretchability, cushioning properties and moisture permeability of the adhesive skin patch, the bubble fraction of the substrate (foam sheet) is 70% or more, preferably 80% or more and more preferably 85% or more.

On the other hand, from the standpoint of securing the strength of the adhesive skin patch, the bubble fraction of the substrate (foam sheet) is preferably 98.5% or less and more preferably 97.5% or less.

In addition, the substrate 2 is formed of a foam sheet, and therefore has excellent stretchability. As a result, the occurrence of peeling, discomfort (skin tightness) and skin irritation (pain) due to expansion and contraction of a skin is suppressed.

The adhesive skin patch of the present embodiment is that only one side thereof may be an adhesive surface and both surfaces thereof may be an adhesive surface. In other words, the adhesive skin patch of the present embodiment may be provided with only an adhesive layer (hereinafter referred to as a "first adhesive layer") partially formed on the first surface of the substrate as an adhesive layer, and may be further provided with another adhesive layer (hereinafter referred to as a "second adhesive layer") formed on the second surface. The second adhesive layer may be partially formed on the second surface of the substrate and may be formed on the entire second surface of the substrate.

As one aspect of the present embodiment, a schematic view showing the state that a wearable device 16 has been mounted on a skin 15 using an adhesive skin patch 11 provided with, as adhesive layers, a first adhesive layer 13 partially formed on a first surface of a substrate 12 and a second adhesive layer 14 formed on a second surface of the substrate, that is, the adhesive skin patch 11, both sides of which being an adhesive surface, is shown in FIG. 2. When the wearable device 16 is mounted on the skin using the adhesive skin patch 11, both sides of which being an adhesive surface, for example, the first adhesive layer 13 partially formed is stuck to the skin 15 and the second adhesive layer 14 is stuck to the wearable device 16.

Thus, when the adhesive skin patch 11 of the present embodiment, both sides of which being an adhesive surface, has been used, the wearable device 16 can be mounted on the skin 15 with particularly difficult peeling and suppression of discomfort and skin irritation as described hereinafter, and this is preferred.

A wearable device does not generally have moisture permeability. Therefore, sweat generated just below the wearable device is difficult to move outside from the surface of the substrate. As a result, stuffiness of a skin surface is remarkable, and this causes peeling, discomfort and skin irritation. On the other hand, when the wearable device 16 has been mounted on a skin using the adhesive skin patch 11 of the present embodiment as shown in FIG. 2, sweat generated just under the wearable device 16 passes through the portion on which the first adhesive layer 13 is not formed and open cells in the foam sheet constituting the substrate 12 and is easy to move outside from the edge of the substrate 12. As a result, peeling, discomfort and skin irritation due to sweat are difficult to occur.

A wearable device is generally hard and cannot be stretched following expansion and contraction of a skin. As a result, peeling, skin tightness and pain due to this are difficult to be suppressed. On the other hand, when the wearable device 16 has been mounted on the skin using the adhesive skin patch 11 of the present embodiment as shown in FIG. 2, the substrate 12 having excellent cushioning properties is present between the skin 15 and the wearable device 16. As a result, stress due to the difference in stretchability between the skin 15 and the wearable device 16 can be relaxed, and peeling, skin tightness and pain due to the difference in stretchability can be suppressed.

When the wearable device is mounted on the skin using the conventional adhesive skin patch so as to cover the wearable device with the conventional adhesive skin patch, the wearable device is directly brought into contact with the skin so as to be pushed to the skin. As a result, impression and skin irritation such as pain occur on the skin. On the other hand, when the wearable device 16 has been mounted on the skin using the adhesive skin patch 11 of the present embodiment as shown in FIG. 2, the wearable device 16 is brought into contact with the skin 15 through the substrate 12 having excellent cushioning properties, and is not pushed on the skin 15. As a result, skin irritation is difficult to occur.

When the wearable device 26 is mounted on the skin using an adhesive skin patch 21, only one side of which being an adhesive surface, that is, the adhesive skin patch 21 that is not provided with the second adhesive layer, it is preferred that a first adhesive layer 23 is stuck to a skin 25 and the wearable device 26 is stuck to a second surface of a substrate 22 using an adhesive or a double-sided tape (not shown), as shown in FIG. 3. By mounting the wearable device on the skin like this, the same effect when the adhesive skin patch, both sides of which being an adhesive surface, is used can be exhibited.

Preferred aspect of the mounting method of the wearable device using the adhesive skin patch of the present embodiment is described above, but uses of the adhesive skin patch of the present embodiment are not limited to this. For example, the wearable device may be mounted on the skin so as to cover the wearable device with the adhesive skin patch of the present embodiment. Alternatively, the adhesive skin patch of the present embodiment can be used to protect a wound, like an adhesive plaster, or can be used to fix a bandage or gauze to the affected area, like a surgical tape.

To improve cushioning properties of the substrate and to make easy to move moisture from the edge of the substrate, the thickness of the substrate is preferably large. Therefore, the thickness of the substrate (foam sheet) is preferably 0.100 mm or more, more preferably 0.150 mm or more and still more preferably 0.200 mm or more.

On the other hand, in case where the thickness of the substrate is too large, there is a possibility that the substrate is easy to break or is easy to shift. Therefore, the thickness of the substrate (foam sheet) is preferably 3.000 mm or less, more preferably 2.000 mm or less and still more preferably 1.000 mm or less.

To improve stretchability, cushioning properties and moisture permeability of the substrate, the density of the substrate is preferably small. Therefore, the density of the substrate (foam sheet) is preferably 0.5 g/cm$^3$ or less, more preferably 0.4 g/cm$^3$ or less and still more preferably 0.35 g/cm$^3$ or less.

On the other hand, the density of the substrate is preferably not too small from the standpoint of securing strength of the adhesive skin patch. Therefore, the density of the substrate (form sheet) is preferably 0.15 g/cm$^3$ or more, more preferably 0.2 g/cm$^3$ or more and still more preferably 0.25 g/cm$^3$ or more.

The stretchability of the substrate can be evaluated by various methods. For example, the stretchability can be evaluated using breaking strength and breaking elongation that are measured by the method described in the item of examples.

From the standpoint of the improvement of stretchability, the breaking strength of the substrate (foam sheet) is preferably 20 N/20 mm or less, more preferably 10 N/20 mm or less and still more preferably 5 N/20 mm or less, and the breaking elongation thereof is preferably 100% or more, more preferably 200% or more and still more preferably 250% or more.

The material of the substrate (foam sheet) is not particularly limited, but is, for example, an amorphous crosslinking type. For example, the material includes polyurethane, polyolefin (such as polyethylene, polypropylene and their copolymers), silicone, acryl and rubber. Of those, synthetic rubber (SBR) and polyurethane are particularly preferred.

The production method of the substrate is not particularly limited, and for example, the substrate can be appropriately produced by the conventional methods.

In the present embodiment, the adhesive layer 3 is partially formed on one side of the substrate 2 in a wavy line shape. A constitution example of the adhesive layer 3 is shown in FIG. 7. In a constitution not encompassed by the present invention, the adhesive layer 3 may be formed on the substrate 2 in a lattice shape as shown in FIG. 4, in a linear shape as shown in FIG. 5, in a dot shape as shown in FIG. 6. In the present invention, a wavy line constitution is used, since in case where the adhesive skin patch 1 of the present embodiment is cut and used, the change with the passage of time by sticking time of a cross-section of a pattern shape is small and the proportion of the area occupied by the adhesive layer 3 in the cut surface is difficult to change even though the cutting place and direction differ. When the proportion of the area occupied by the adhesive layer 3 in the cut surface is difficult to change, adhesive force in the vicinity of the cut portion (edge of adhesive skin patch 1) is difficult to change, and as a result, the occurrence of peeling from the edge can be stably suppressed. In other words, by forming the adhesive layer 3 in a wavy line shape, the occurrence of peeling from the edge can be stably suppressed without being influenced by the place or direction cutting the adhesive skin patch 1 of the present embodiment. The form of the wavy line shape may be a wavy line shape that is symmetrically formed at both sides of a base line, such as sine curve, pseudo sine curve, triangular wave or arc wave, and may be a wavy line shape that proceeds in arc, triangle, ellipse or other shapes at only one side of a base line. Line width of pattern shape, interval (pitch) between line and line, amplitude, period and the like can be appropriately set depending on the purpose of use.

The conventional adhesives that can be used for sticking to a skin such as an acrylic adhesive, a rubber adhesive, a urethane adhesive or a silicone resin can be used as the adhesive forming the adhesive layer (first adhesive layer) that is stuck to a skin without particular limitation. Above all, an adhesive containing an acrylic copolymer, that is, an acrylic adhesive, is preferred from that adhesive force to a skin is easily adjusted and skin irritation is less.

The acrylic copolymer is not particularly limited, but for example, an acrylic copolymer obtained from a monomer mixture containing a (meth)acrylic acid alkyl ester monomer and an alkoxy group-containing ethylenically unsaturated monomer is preferably used.

The (meth)acrylic acid alkyl ester monomer is a main component that imparts good skin stickability to the adhesive layer, and in particular, use of a long chain alkyl group having 6 or more carbon atoms, preferably 6 to 18 carbon atoms, in the alkyl moiety is effective. The (meth)acrylic acid alkyl ester monomer has the advantages that irritation to a skin is relatively small and the decrease of adhesive force is difficult to occur even by the use of a long period of time.

The (meth)acrylic acid alkyl ester monomer includes butyl ester, propyl ester, octyl ester, nonyl ester, decyl ester, dodecyl ester, lauryl ester and the like of acrylic acid or methacrylic acid, and those can be used in one kind or by combining two or more kinds. The alkyl ester chain thereof may be a straight chain and may be a branched chain.

The alkoxy group-containing ethylenically unsaturated monomer is a component that imparts water vapor permeability, so-called moisture permeability, to the acrylic copolymer. The alkoxy group-containing ethylenically unsaturated monomer is not particularly limited, but is preferably, for example, alkoxyalkyl acrylate having an alkoxy group with 1 to 4 carbon atoms, such as methoxypolyethylene glycol acrylate, ethoxydiethylene glycol acrylate, butoxydiethylene glycol acrylate, methoxyethyl acrylate, ethoxyethyl acrylate or butoxyethyl acrylate.

The acrylic copolymer is preferably an acrylic copolymer obtained from a monomer mixture containing a carboxyl group-containing ethylenically unsaturated monomer. The carboxyl group-containing ethylenically unsaturated monomer is a component that improves cohesive force of a copolymer obtained, and is useful in adjusting characteristics of the adhesive layer. Representative examples of the monomer include acrylic acid, itaconic acid, crotonic acid, fumaric acid and (anhydrous) maleic acid. Of those, acrylic acid is exemplified as the preferable monomer in the points of copolymerizability, handleability and the like.

The acrylic copolymer constituting the adhesive is preferably an acrylic copolymer obtained by copolymerization of the (meth)acrylic acid alkyl ester monomer and the alkoxy group-containing ethylenically unsaturated monomer and more preferably an acrylic copolymer obtained by copolymerization of the (meth)acrylic acid alkyl ester monomer, the alkoxy group-containing ethylenically unsaturated monomer and the carboxyl group-containing ethylenically unsaturated monomer.

In the acrylic copolymer, as necessary, in addition to the above-mentioned respective monomers, a monomer such as styrene, vinyl acetate or N-vinyl-2-pyrrolidone may be appropriately copolymerized as a modifier resin for performing various modifications such as incorporation of hydrophilicity.

The content of the (meth)acrylic acid alkyl ester monomer in the acrylic copolymer is preferably 40 to 80 mass % and more preferably 50 to 75 mass %. When the content of the (meth)acrylic acid alkyl ester monomer is in the above-mentioned range, the adhesive layer obtained shows good skin stickability, does not generate the phenomenon that an adhesive remains on the skin surface when peeling away from the skin, and has excellent peelability.

The content of the alkoxy group-containing ethylenically unsaturated monomer in the acrylic copolymer is preferably 10 to 60 mass %, more preferably 20 to 60 mass % and still more preferably 25 to 50 mass %. When the content of the alkoxy group-containing ethylenically unsaturated monomer is in the above-mentioned range, the resulting copolymer can impart excellent moisture permeability to the adhesive layer.

When the carboxyl group-containing ethylenically unsaturated monomer is contained, the content of the alkoxy group-containing ethylenically unsaturated monomer is preferably 10 to 50 mass % and more preferably 20 to 45 mass %.

The content of the carboxyl group-containing ethylenically unsaturated monomer is preferably 1 to 10 mass % and more preferably 3 to 8 mass %. When the content of the carboxyl group-containing ethylenically unsaturated monomer is in the above-mentioned range, the resulting copolymer can impart excellent cohesive force to the adhesive layer, can suppress the phenomenon of adhesive residue and additionally can suppress skin irritation.

Weight average molecular weight of a soluble portion in a solvent for molecular weight measurement of the acrylic copolymer is preferably 500,000 to 2,500,000 and more preferably about 600,000 to 2,000,000.

The weight average molecular weight used herein is a value measured using gel permeation chromatography (GPC). A sample for measurement is dissolved in tetrahydrofuran, a soluble portion passing through a 0.45 $\mu m\phi$ membrane filter is subjected to GPC measurement and the value is calculated in terms of polystyrene.

The adhesive layer (first adhesive layer) that is stuck to a skin preferably contains a liquid or pasty component at room temperature (25°C). The liquid or pasty component at room temperature is a component for imparting flexibility to the adhesive layer and reducing skin irritation when the adhesive layer has been brought into contact with the skin.

The liquid or pasty component at room temperature is not particularly limited so long as the component is compatible with the acrylic copolymer. Examples of the component that can be used include esters of phthalic acid, maleic acid, adipic acid, stearic acid or various fatty acids and alkyl alcohols, and esters of those acids and polyhydric alcohols such as ethylene glycol, glycerin or sorbitan. More specifically, examples of a monohydric alcohol ester include dibutyl phthalate, di-2-ethlhexyl phthalate, dibutyl adipate, di-2-ethylhexyl sebacate, dibutyl maleate, ethyl myristate, isopropyl myristate, isopropyl palmitate, butyl stearate, isopropyl isostearate, hexyl laurate, cetyl lactate, myristyl laurate, diethyl phthalate, octyldodecyl myristate, octyldodecyl oleate, hexyldecyl dimethyloctanoate, cetyl 2-ethylhexanoate, isocetyl 2-ethylhexanoate, stearyl 2-ethylhexanoate and dioctyl succinate. Examples of a dihydric or higher alcohol ester include propylene glycol dicaprylate, propylene glycol dicaprate, propylene glycol diisostearate, glyceryl monocaprylate, glyceryl tricaprylate, glyceryl tri-2-ethylhexanoate, glyceryl tricaprate, glyceryl trilaurate, glyceryl triisostearate, glyceryl trioleate, sorbitan trioeleate and trimethylolpropane tri-2-ethylheanoate. Those esters can be used in one kind or as mixtures of two or more kinds. From the standpoint of compatibility with the acrylic copolymer, the liquid or pasty component at room temperature is preferably carboxylic acid ester, more preferably glycerin fatty acid ester and still more preferably glycerin ester of saturated fatty acid.

Examples of the fatty acid include saturated fatty acids such as caprylic acid, capric acid and 2-ethylhexanoic acid. Those acids can be used alone or as combinations of two or more thereof. Those saturated fatty acids do not have an unsaturated double bond and therefore can prevent oxidative deterioration of the adhesive, that is, the adhesive layer.

When the above-described caprylic acid and the like are used as saturated fatty acids, glyceryl tricaplyrate, glyceryl tricaprate and glyceryl tri-2-ethylhexanoate are obtained as esters of various saturated fatty acids and alcohols. Of those carboxylic acid esters, glyceryl tricaprylate is particularly preferably used from the standpoint of compatibility with the acrylic copolymer, and the like.

The content of the liquid or pasty component at room temperature is, for example, preferably 10 to 100 parts by mass per 100 parts by mass of the acrylic copolymer. When the content of the component is in the above range, sufficient flexibility can be imparted to the adhesive layer. As a result, the adhesive layer has good stickability to a skin, suppresses physical skin irritation when peeling the patch from the skin and can achieve good peelability.

To improve adhesive force to a skin, the adhesive layer (first adhesive layer) that is stuck to the skin preferably further contains a tackifier. Rosin type tackifier, terpene type tackifier and the like can be used as the tackifier. Softening point of the tackifier is preferably 60°C or higher, more preferably 80°C or more and still more preferably 100°C or higher.

The content of the tackifier is, for example, preferably 5 to 50 parts by mass, more preferably 5 to 30 parts by mass and still more preferably 5 to 20 parts by mass, per 100 parts by mass of the acrylic copolymer.

As necessary, various additives such as the conventional plasticizer, softener, filler, stabilizer, antioxidant, antibacterial agent and fungicide may be appropriately added to the adhesive layer (first adhesive layer) that is stuck to a skin in a range that does not deviate the object of the present invention.

The adhesive layer (first adhesive layer) that is stuck to a skin is preferably subjected to a crosslinking treatment in order to impart appropriate cohesive force to the adhesive layer. The crosslinking treatment includes physical crosslinking by irradiation with ionizing radiation such as electron beams, $\gamma$ rays or X rays, and chemical crosslinking by a crosslinking agent. From the standpoints of handling properties and good reproducibility of crosslinking, the adhesive layer is preferably subjected to the chemical crosslinking treatment by a crosslinking agent. For example, a crosslinking agent is added to an adhesive composition having materials constituting an adhesive layer added thereto, and the resulting adhesive composition is heat-treated to perform a crosslinking treatment. Examples of such a crosslinking agent include crosslinking agents used in conducting a crosslinking treatment, such as an isocyanate crosslinking agent, a peroxide crosslinking agent or a metal chelate crosslinking agent.

The adhesive layer having been subjected to the crosslinking treatment as above holds a balance between appropriate skin stickability and cohesive force by the degree of crosslinking, but a molecular weight and molecular weight distribution of the acrylic copolymer also affect the adjustment of those.

The adhesive layer (first adhesive layer) that is stuck to a skin is preferably thick from the standpoint of improvement of adhesive force. However, when too thick, the edge of the adhesive layer is caught and is easy to be peeled or the adhesive layer is easy to protrude from the edge and the edge is easy to be contaminated, during the adhesive layer being stuck to a skin.

For the above reasons, the thickness of the adhesive layer (first adhesive layer) is preferably 50 $\mu$m or more and more preferably 60 $\mu$m or more. On the other hand, the thickness is preferably 120 $\mu$m or less and more preferably 100 $\mu$m or less.

In case where the adhesive skin patch of the present embodiment has the adhesive layer (first adhesive layer) partially formed on one side (first surface) of the substrate and another adhesive layer (second adhesive layer) formed on the other side (second surface) of the substrate, the thickness of the second adhesive layer is not particularly limited, but is, for example, 30 to 80 $\mu$m and preferably 40 to 70 $\mu$m.

In this case, the second adhesive layer may be partially formed on the second surface of the substrate or may be formed on the whole of the second surface of the substrate.

For example, an acrylic adhesive, a rubber adhesive, a urethane adhesive, a silicone adhesive and the like that are exemplified as the adhesive forming the first adhesive layer can be used as the adhesive forming the second adhesive layer, but the adhesive forming the second adhesive is not limited to those adhesives.

The adhesive skin patch of the present embodiment may be provided with the substrate described above and a layer other than the adhesive layer so long as the effect of the present invention is exerted.

In the adhesive skin patch of the present embodiment, the surface of the adhesive layer is preferably protected until using the adhesive skin patch for the purpose of preventing the surface of the adhesive layer from being contaminated. The protection method of the adhesive includes a method of protecting the adhesive by laminating a release liner on the adhesive layer. In case where only one side of the adhesive skin patch is an adhesive surface, the adhesive layer is protected by that the surface of the substrate on which the adhesive layer is not formed is subjected to a release treatment and the adhesive skin patch is wound such that the adhesive layer is laminated on the release-treated surface. In addition, in case where both sides of the adhesive skin patch are an adhesive surface, both adhesive layers may be protected by that one release-treated surface of a release liner, both sides of which having a release-treated surface, is laminated on one adhesive surface and the adhesive skin patch is wound such that the other release-treated surface of the release liner is brought into contact with the other adhesive surface.

<Production method>

Production method of the adhesive skin patch of the present embodiment is not particularly limited. For example, the adhesive layer and the substrate are separately formed, and then those are laminated to each other. Alternatively, a solution of the adhesive is applied to the substrate and dried, thereby forming the adhesive layer.

The adhesive layer may be formed on only a part of the surface of the substrate from the first to form a partial adhesive layer, and the adhesive layer may be formed on the whole of the substrate from the first and the adhesive layer is then partially removed to form a partial adhesive layer.

Examples

The effects of the present invention are described in detail below by reference to examples and comparative examples, but the present invention is not construed as being limited to those. All "parts" and "%" in the following examples and comparative examples mean "parts by mass" and "mass %", respectively.

[Production of substrate]

(Substrate 1)

2.88 g of a vulcanization accelerator (trade name: CLFB-2, manufactured by E-TEC) was added to 75 g of a styrene-butadiene copolymer mixture (trade name: AH748-M4, manufactured by E-TEC), the resulting mixture was stirred in a rotational speed of 1800 rpm for 5 minutes using a stirrer. The solution thus obtained was applied to the release-treated surface of a release-treated polyethylene terephthalate film (trade name: DIAFOII, MRF38, manufactured by Mitsubishi Chemical Corporation) such that the thickness after drying became 0.325 mm, and then dried at 130°C for 10 minutes. The coated surface was subjected to a plasma treatment to obtain a substrate 1. The substrate 1 was a porous sheet having open cells.

(Substrate 2)

A polyurethane foam sheet (trade name: HYDROPHILIC FORM1012, manufactured by Freudenberg) was prepared as a substrate 2. The substrate 2 was a porous sheet having open cells.

(Substrate 3)

A polypropylene foam sheet (trade name: FOLEC OPN 1.0t, manufactured by INOAC Corporation) was prepared as a substrate 3. The substrate 3 was a porous sheet having open cells.

(Substrate 4)

A substrate 4 was obtained in the same manner as the substrate 1, except that 3.84 g of a vulcanization accelerator (trade name: CLFB-2, manufactured by E-TEC) was added to a 100 g styrene-butadiene copolymer mixture (trade name: AH748-M4, manufactured by E-TEC). The substrate 4 was a porous sheet having open cells.

(Substrate 5)

A polyethylene foam sheet (trade name: VOLARA XL-H #03001 (80), manufactured by Sekisui Chemical Co., Ltd.) was subjected to a corona treatment to obtain a substrate 5. The substrate 5 was a porous sheet that does not have open cells and has only closed cells.

(Substrate 6)

A polyethylene terephthalate nonwoven fabric sheet (trade name: SONTARA #8010, manufactured by Du Pont) was prepared as a substrate 6.

(Substrate 7)

A polyurethane film (trade name: ESMER URS-210, manufactured by Nihon Matai Co., Ltd.) was subjected to a corona treatment to obtain a substrate 7.

[Evaluation of substrate]

Thickness, density, bubble fraction, breaking strength and breaking elongation of the substrates 1 to 7 obtained above were measured as follows.

(Thickness)

Thicknesses at 5 points of each substrate were measured using a dial gauge, and an average value of the measurement results was used as a thickness of each substrate.

(Density)

Each substrate was cut into a size of 5 cm × 5 cm, and its weight was measured. Density was calculated by the following formula using the weight measured and the above thickness.

$$\text{Density (g/cm}^3) = \text{Weight (g)}/(5 \times 5 \times \text{thickness (cm}^3))$$

(Bubble fraction)

Bubble fraction of each substrate was calculated by the following formula.

$$\text{Bubble fraction (\%)} = (1 \times \text{specific gravity of substrate material} - \text{density}) \times 100$$

(Breaking strength and breaking elongation)

Each substrate was cut into a size of 20 mm width and 100 mm length, set up on a tensile tester (trade name: AUTOGRAPH AG-IS, manufactured by Shimadzu Corporation) in a chuck distance of 40 mm and pulled in a tensile speed of 300 mm/min. Load and elongation when the substrate broke were measured. The same measurement was conducted three times, and its average value was used as breaking strength and breaking elongation of each substrate.

[Production of adhesive skin patch]

(Example 1)

20 parts of glyceryl tricaprylate, 10 parts of a rosin ester (HARITACK PCJ, manufactured by Harima Chemicals Group, Inc.) as a tackifier and 0.07 parts of a trifunctional isocyanate compound (trade name: CORONATE HL, manufactured by Tosoh Corporation) as a crosslinking agent were blended with 100 parts of an acrylic copolymer (copolymer comprising isononyl acrylate : 2-methoxyethyl acrylate: acrylic acid = 65 parts : 30 parts : 5 parts) in toluene, and a uniform adhesive solution was prepared.

The adhesive solution was applied to a release-treated surface of a release paper (trade name: KP-64 SHIROMARU D MARUMIZU, manufactured by LINTEC Corporation) such that a thickness after drying became 70 μm, and a wavy groove of width: 0.5 mm, amplitude: 50 mm, period: 140 mm and pitch: 5 mm was provided, followed by drying at 130°C for 3 minutes. Thus, an adhesive layer was formed. The adhesive layer formed was transferred to and laminated on a plasma-treated surface of the substrate 1, followed by allowing to stand at 60°C for 72 hours. Thus, an adhesive skin patch of Example 1 was obtained.

(Examples 2 and 3 and Comparative Examples 1 to 5)

Adhesive skin patch of Example 2 was obtained in the same manner as in Example 1, except that the substrate 2 was used in place of the substrate 1.

Adhesive skin patch of Example 3 was obtained in the same manner as in Example 1, except that the substrate 3 was used in place of the substrate 1.

Adhesive skin patch of Comparative Example 1 was obtained in the same manner as in Example 1, except that the substrate 4 was used in place of the substrate 1.

Adhesive skin patch of Comparative Example 2 was obtained in the same manner as in Example 1, except that the substrate 5 was used in place of the substrate 1.

Adhesive skin patch of Comparative Example 3 was obtained in the same manner as in Example 1, except that a groove was not provided on the adhesive layer.

Adhesive skin patch of Comparative Example 4 was obtained in the same manner as in Example 1, except that the substrate 6 was used in place of the substrate 1.

Adhesive skin patch of Comparative Example 5 was obtained in the same manner as in Example 1, except that the substrate 7 was used in place of the substrate 1.

[Evaluation of adhesive skin patch]

Moisture decrease rate of the adhesive skin patch of each example and comparative example was measured as follows. Additionally, feeling of sticking was evaluated.

(Moisture decrease rate)

The adhesive skin patch of each example and comparative example was cut into a size of 30 mm × 30 mm, and a test sample was produced.

Collagen film was dipped in purified water for 10 minutes and taken out. Water on the surface of the film was wiped off and the film was stuck to a bakelite plate by a double-sided tape. The amount of moisture on the surface of the collagen film just after sticking to the bakelite was measured and the value was used as an initial moisture percentage.

Next, the adhesive layer of the test sample was stuck to the collagen film, allowed to stand at 23°C for 8 hours, and then peeled. After peeling, the amount of moisture in the portion of the collagen film to which the test sample was stuck was measured, and the value was used as moisture amount after storage.

Corneometer CM825 (manufactured by Integral Corporation) was used for the measurement of moisture amount. Hand roller was used in sticking the collagen film to the bakelite plate and in sticking the collagen film to the test sample.

The moisture decrease rate was calculated by the following formula (1) using the initial moisture amount and the moisture amount after storage.

$$\text{Moisture decrease percentage}(\%) = [(\text{initial moisture amount} - \text{moisture amount after storage})/\text{initial moisture amount}] \times 100 \qquad (1)$$

(Feeling of sticking)

Adhesive skin patch of each example and comparative example was cut into a size of 10 mm × 50 mm, and a test sample was produced.

The adhesive layer of the test sample from which a release paper had been peeled was stuck to a joint part of an index finger of a participant. The participant bent his index finger and the feeling of sticking of the patch was evaluated by the following criteria.

5: No feeling of tightness
4: Feeling of tightness in a degree that patch is not annoying
3: Feeling of tightness in a degree that patch is slightly annoying, but participant can live life
2: Feeling of tightness in a degree that patch is annoying, but participant can endure and live life without peeling patch
1: Feeling of tightness in a degree that participant cannot endure without peeling patch

[Table 1]

| | | Example 1 | Example 2 | Example 3 | Comparative Example 1 | Comparative Example 2 | Comparative Example 3 | Comparative Example 4 | Comparative Example 5 |
|---|---|---|---|---|---|---|---|---|---|
| Substrate | Material | SBR | PU | PP | SBR | PE | SBR | PET | PU |
| | Form | Foam | Foam | Foam | Foam | Foam | Foam | Non-woven fabric | Film |
| | Open cell | Presence | Presence | Presence | Presence | None | Presence | None | None |
| | Thickness (mm) | 0.325 | 1.36 | 0.920 | 0.352 | 0.110 | 0.325 | 0.280 | 0.030 |
| | Density (g/cm$^3$) | 0.257 | 0.120 | 0.035 | 0.318 | 0.271 | 0.257 | 0.156 | 1.220 |
| | Bubble Fraction | 74% | 88% | 97% | 68% | 73% | 74% | 84% | 0% |
| | Breaking strength (N/20 mm) | 1.34 | 1.4 | 4.40 | 1.50 | 9.50 | 1.34 | 40.7 | 18.7 |
| | Breaking elongation (%) | 374 | 273 | 110 | 342 | 480 | 374 | 57 | 1019 |
| Adhesive layer | Shape | Wavy line Shape | Wavy line shape | Wavy line shape | Wavy line shape | Wavy line shape | Entire surface | Wavy line shape | Wavy line shape |
| Evaluation of patch | Moisture decrease rate (%) | 63.2 | 86.7 | 87.1 | 52.6 | 30.4 | 21.1 | 60.0 | 15.4 |
| | Feeling of Sticking | 5 | 5 | 5 | 5 | 4 | 5 | 2 | 5 |

SBR: Styrene-butadiene rubber
PU: Polyurethane
PP: Polypropylene
PE: Polyethylene
PET: Polyethylene terephthalate

EP 3 919 032 B1

In Comparative Example 2 in which the foam sheet that do not have open cells was used, the moisture decrease rate was small. Therefore, the adhesive skin patch of Comparative Example 2 is easy to cause peeling, discomfort and skin irritation by sweat.

Even though the foam sheet having open cells was used as the substrate, Comparative Example 3 in which the adhesive layer was formed on the entire surface of one side of the substrate was that the moisture decrease rate was particularly small. Therefore, the adhesive skin patch of Comparative Example 3 is particularly easy to cause peeling, discomfort and skin irritation by sweat.

In Comparative Example 1 in which the foam sheet having open cells but having the bubble fraction of less than 70% was used, the moisture decrease rate was not sufficient. Therefore, the adhesive skin patch of Comparative Example 1 may cause peeling, discomfort and skin irritation by sweat.

In Comparative Example 4 in which non-woven fabric, not foam sheet, was used as the substrate, the evaluation result of feeling of sticking was poor. Therefore, the adhesive skin patch of Comparative Example 4 is easy to cause peeling, discomfort and skin irritation by expansion and contraction of a skin.

In Comparative Example 5 in which urethane film, not foam sheet, was used as the substrate, the moisture decrease rate was particularly small. Therefore, the adhesive skin patch of Comparative Example 5 is particularly easy to cause peeling, discomfort and skin irritation by sweat.

On the other hand, in Examples 1 to 3, the moisture decrease rate was large and the evaluation result of the feeling of sticking was good. Therefore, the adhesive skin patches of Examples 1 to 3 are difficult to cause peeling, discomfort and skin irritation by sweat or expansion and contraction of a skin.

Although preferred embodiments of the present invention have been described, the present invention is not limited to the embodiments described above, and various modifications or substitutions can be added to the embodiments described above in the scope that the scope of the present invention does not deviate.

Reference Signs List

1, 11, 21: Adhesive skin patch
2, 12, 22: Substrate
3, 13, 23: Adhesive layer (first adhesive layer)
14: Other adhesive layer (second adhesive layer)
15, 25: Skin
16,26: Wearable device

**Claims**

1. An adhesive skin patch comprising a substrate and an adhesive layer,

   wherein the substrate is formed of a foam sheet having open cells and a bubble fraction of 70% or more, and the adhesive layer is partially formed on one side of the substrate in a wavy line shape.

2. The adhesive skin patch according to claim 1, wherein another adhesive layer is further provided on a surface on a side opposite the surface of the substrate having the adhesive layer.

3. The adhesive skin patch according to claim 1 or 2, wherein the adhesive layer contains an acrylic copolymer.

4. The adhesive skin patch according to claim 3, wherein the acrylic copolymer is an acrylic copolymer obtained by copolymerizing a monomer mixture containing a (meth)acrylic acid alkyl ester monomer and an alkoxy group-containing ethylenically unsaturated monomer.

5. The adhesive skin patch according to any one of claims 1 to 4, wherein a bubble fraction of the foam sheet is 70 to 98.5%.

6. The adhesive skin patch according to claim 1, wherein a density of the substrate is 0.2 g/cm$^3$ or more.

7. The adhesive skin patch according to claim 3, wherein the adhesive layer contains a rosin type tackifier or a terpene type tackifier.

**Patentansprüche**

1. Klebepflaster für die Haut, umfassend ein Substrat und eine Klebeschicht,

    wobei das Substrat aus einer Schaumstofffolie mit offenen Zellen und einem Blasenanteil von 70 % oder mehr gebildet ist, und
    die Klebeschicht teilweise auf einer Seite des Substrats in Wellenlinienform aufgebracht ist.

2. Klebepflaster für die Haut nach Anspruch 1, worin zusätzlich eine weitere Klebstoffschicht auf der Oberfläche auf der Seite des Substrats aufgebracht ist, die der Substratoberfläche mit der Klebstoffschicht gegenüberliegt.

3. Klebepflaster für die Haut nach Anspruch 1 oder 2, wobei die Klebeschicht ein Acryl-Copolymer enthält.

4. Klebepflaster für die Haut nach Anspruch 3, wobei das Acryl-Copolymer ein Acryl-Copolymer ist, das durch Copolymerisation einer Monomermischung erhalten wird, die ein (Meth)acrylsäure-Alkylester-Monomer und ein Alkoxygruppen enthaltendes ethylenisch ungesättigtes Monomer enthält.

5. Klebepflaster für die Haut nach einem der Ansprüche 1 bis 4, wobei der Blasenanteil der Schaumstofffolie 70 bis 98,5 % beträgt.

6. Klebepflaster für die Haut nach Anspruch 1, wobei die Dichte des Substrats 0,2 g/cm$^3$ oder mehr beträgt.

7. Klebepflaster für die Haut nach Anspruch 3, wobei die Klebeschicht einen Klebrigmacher vom Kolophoniumtyp oder einen Klebrigmacher vom Terpentyp enthält.


**Revendications**

1. Timbre adhésif cutané comprenant un substrat et une couche adhésive,

    dans lequel le substrat est formé d'une feuille expansée ayant des alvéoles ouvertes et une fraction bulle de 70 % ou plus, et
    la couche adhésive est partiellement formée sur un côté du substrat en une forme de ligne ondulée.

2. Timbre adhésif cutané selon la revendication 1, dans lequel une autre couche adhésive est fournie en outre sur une surface sur un côté opposé à la surface du substrat ayant la couche adhésive.

3. Timbre adhésif cutané selon la revendication 1 ou 2, dans lequel la couche adhésive contient un copolymère acrylique.

4. Timbre adhésif cutané selon la revendication 3, dans lequel le copolymère acrylique est un copolymère acrylique obtenu par copolymérisation d'un mélange de monomères contenant un monomère d'ester alkylique d'acide (méth)acrylique et un monomère éthyléniquement insaturé contenant un groupe alcoxy.

5. Timbre adhésif cutané selon l'une quelconque des revendications 1 à 4, dans lequel une fraction bulle de la feuille expansée est de 70 à 98,5 %.

6. Timbre adhésif cutané selon la revendication 1, dans lequel une densité du substrat est de 0,2 g/cm$^3$ ou plus.

7. Timbre adhésif cutané selon la revendication 3, dans lequel la couche adhésive contient un agent tackifiant type colophane ou un agent tackifiant type terpène.

*FIG. 1*

1

2

3  3  3  3  3  3  3  3

*FIG. 2*

16
14
12  } 11
13
15

*FIG. 3*

26
22 } 21
23
25

FIG. 4

FIG. 5

FIG. 6

3    2

*FIG. 7*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015530225 T
- DE 102016008257 A1
- WO 2018030447 A1
- EP 0106440 A1
- JP H045807 U